# EUROPEAN PATENT SPECIFICATION

(11) **EP 4 414 683 B1**
(45) Date of publication and mention of the grant of the patent: **21.01.2026**
(21) Application number: 23846927.4
(22) Date of filing: 21.07.2023
(51) Int. Cl.: G01N 1/22, H01M 10/48, G01N 33/00

(54) **GAS COLLECTION DEVICE**
GASSAMMELVORRICHTUNG
DISPOSITIF DE COLLECTE DE GAZ

(30) Priority: 28.07.2022 KR 20220094123
(43) Date of publication of application: 14.08.2024
(73) Proprietor: LG Energy Solution, Ltd., Seoul 07335 (KR)
(72) Inventor: KIM, Kyung Min, Daejeon 34122 (KR); CHOI, Nak Hee, Daejeon 34122 (KR)
(74) Representative: Goddar, Heinz J.
(86) International application number: PCT/KR2023/010584
(87) International publication number: WO 2024/025268

(56) References cited:
- EP-A1- 4 397 953
- EP-A2- 3 736 903
- CN-U- 212 722 245
- JP-A- 2002 313 437
- JP-A- 2006 226 745
- JP-A- 2017 075 655
- JP-B2- 4 517 877
- KR-A- 20190 125 921
- KR-A- 20190 125 921
- KR-Y1- 200 475 466

## Description

### Technical Field

The present disclosure relates to a gas collecting apparatus, and particularly to a gas collecting apparatus capable of providing an optimized gas concentration to an analysis device by controlling a space in which gas generated in a battery is diffused in a process of collecting the gas.

### Background Art

In general, a secondary battery is a battery which can be used repeatedly through a discharging process in which chemical energy is converted into electrical energy and a charging process in the opposite direction to the discharging process and which includes nickelcadmium (Ni-Cd) batteries, nickel-metal hydride (Ni-MH) batteries, lithium-metal batteries, lithium-ion (Li-ion) batteries, and lithium-ion polymer batteries. Among these secondary batteries, lithium secondary batteries having high energy density and voltage, long cycle life, and a low self-discharge rate have been commercialized and widely used.

Depending on the reaction within the lithium secondary battery, various types of gases such as hydrogen, oxygen, nitrogen, carbon monoxide, carbon dioxide, hydrocarbons of CₙH₂ₙ₋₂ (n=2~5), CₙH₂ₙ (n=2~5), CₙH₂ₙ₊₂ (n=1~5), and other organic gas species, may be generated.

In addition, the lithium secondary battery degrades while generating a large amount of gas due to electrolyte decomposition as repeated charging and discharging progresses, and this aspect appears differently depending on the design and use form of the battery. Therefore, it is essential to infer a deterioration mechanism of a battery by analyzing gas generated inside the battery during a battery development process.

Therefore, it is very important to collect and accurately analyze the gas generated in the secondary battery. Various gases are generated during the operation of lithium ion batteries, and information on the composition and content of generated gases is useful for developing battery materials, optimizing battery manufacturing processes, and identifying causes of battery defects. To this end, it is important to develop a technology to collect the gas generated inside the secondary battery.

As one method for collecting gas generated from the secondary battery, after placing the secondary battery in an airtight diffusion space and depressurizing the secondary battery, a hole was made in the secondary battery to diffuse the generated gas into the diffusion space accommodating the secondary battery, and samples were taken.

Specifically, a gas collecting container or a gas analyzer in which a vacuum was formed was connected to the diffusion space, and the gas diffused in the diffusion space was delivered to the gas collecting container or the gas analyzer. Accordingly, the concentration of the finally collected gas varied depending on the size of the diffusion space or the specification (size) of the battery.

JP002313437 discloses a gas collection apparatus in which the internal volume of the dilution container is set according to the assumed value of the amount of gas contained in each closed battery to be measured.

Since the concentration of the collected gas not only affects detection sensitivity of the analysis device but is also an important variable for calculating quantitative data, a method for precisely controlling it is required.

### Disclosure of the Invention

### Technical Goals

The present disclosure relates to a gas collecting apparatus, and is to provide a gas collecting apparatus capable of providing an optimized gas concentration to an analysis device by controlling a space in which gas generated in a battery is diffused in the process of collecting the gas.

Technical objects to be achieved by the present disclosure are not limited to the technical objects mentioned above, and other technical objects not mentioned will be clearly understood by those skilled in the art from the description below.

### Technical Solutions

A gas collecting apparatus of the present disclosure may include:
a gas diffusion chamber provided with a gas diffusion space inside;
a variable dilution tank connected to the gas diffusion chamber and provided with a dilution space inside;
a volume control unit configured to control a volume of the dilution space of the variable dilution tank;
a punching unit configured to form a perforated hole in a case of a battery accommodated in the gas diffusion space of the gas diffusion chamber; and
a gas delivery flow path connected to the gas diffusion chamber to receive gas diffused in the gas diffusion space and deliver the gas to a gas collecting container or a gas analyzer, wherein the volume control unit is configured to be operated to expand the dilution space of the variable dilution tank when a pressure of the gas diffusion space is greater than a set pressure.

### Advantageous Effects

A gas collecting apparatus of the present disclosure may be capable of collecting a gas with a constant concentration in a gas collecting container by providing a controllable variable space to a gas diffusion space.

The gas collecting apparatus of the present disclosure may provide an optimized gas concentration to an analysis device by controlling a space in which gas generated from a battery is diffused in the process of collecting the gas.

### Brief Description of Drawings

FIG. 1 is a perspective view illustrating a gas collecting apparatus according of the present disclosure.
FIG. 2 is a perspective view illustrating a state in which a gas diffusion chamber is opened.
FIG. 3 is a perspective view illustrating a variable dilution tank and a volume control unit.
FIG. 4a is a cross-sectional view illustrating a cross section A-A' of FIG. 3.
FIG. 4b is a cross-sectional view illustrating a state in which a dilution space is expanded.
FIG. 5 is a perspective view illustrating a punching unit.

### Best Mode for Carrying Out the Invention

A gas collecting apparatus of the present disclosure may include:
a gas diffusion chamber provided with a gas diffusion space inside;
a variable dilution tank connected to the gas diffusion chamber and provided with a dilution space inside;
a volume control unit configured to control a volume of the dilution space of the variable dilution tank;
a punching unit configured to form a perforated hole in a case of a battery accommodated in the gas diffusion space of the gas diffusion chamber; and
a gas delivery flow path connected to the gas diffusion chamber to receive gas diffused in the gas diffusion space and deliver the gas to a gas collecting container or a gas analyzer.

In the gas collecting apparatus of the present disclosure, the volume control unit may include a moving unit which is located inside the variable dilution tank and moves in a vertical direction, a shaft which is coupled to an upper surface of the moving unit in a state in which a lower end is inserted inside the variable dilution tank through a penetration hole provided on an upper surface of the variable dilution tank and moves in the vertical direction, and a first driving unit configured to rotate the shaft with the vertical direction as a rotation axis.

**In** the gas collecting apparatus of the present disclosure, the gas diffusion chamber may include a lower jig with a battery accommodating groove formed on an upper surface as the gas diffusion space, and an upper jig coupled to an upper surface of the lower jig while covering the battery accommodating groove, wherein a first hole is formed on an upper surface of the upper jig, a lower end of the variable dilution tank is coupled to the upper surface of the upper jig while covering the first hole, and gas in the gas diffusion space and gas in the dilution space are mutually ventilated through the first hole.

**In** the gas collecting apparatus of the present disclosure, the volume control unit may further include a support unit configured to fix a relative distance between the first driving unit and the upper jig.

**In** the gas collecting apparatus of the present disclosure, the volume control unit may further include a measuring unit configured to measure a moving distance of the moving unit in the vertical direction.

**In** the gas collecting apparatus of the present disclosure, the measuring unit may include a moving member configured to move in the vertical direction along with the shaft, a fixing member coupled to the upper surface of the upper jig, and a display member configured to indicate displacement between the moving member and the fixing member.

In the gas collecting apparatus of the present disclosure, an inner space of the variable dilution tank may be provided in a column shape extending in the vertical direction, a side surface of the moving unit may be in close contact with an inner circumferential surface of the variable dilution tank, and the dilution space may be a space located at a lower side of the moving unit in the inner space of the variable dilution tank.

In the gas collecting apparatus of the present disclosure, a sealing cover surrounding the shaft may be provided at an upper side of the moving unit in the inner space of the variable dilution tank, and the sealing cover may be contracted or expanded in the vertical direction.

In the gas collecting apparatus of the present disclosure, an upper end of the sealing cover may be coupled to a ceiling surface of the variable dilution tank in the inner space of the variable dilution tank, and a lower end of the sealing cover may be coupled to the upper surface of the moving unit.

In the gas collecting apparatus of the present disclosure, a second hole for inserting a punching needle into the gas diffusion space may be formed on the upper surface of the upper jig, and the punching unit may include a punching needle housing coupled to the upper surface of the upper jig with a lower end covering the second hole and configured to accommodate the punching needle inside, a rod with a lower end coupled to the punching needle inside the punching needle housing, a second driving unit coupled to an upper end of the rod and configured to move the rod in the vertical direction, and a fixing unit configured to fix a position between the punching needle housing and the second driving unit.

### Modes for Carrying Out the Invention

Hereinafter, embodiments according to the present disclosure will be described in detail with reference to the accompanying drawings. In this process, the size or shape of the components shown in the drawings may be exaggerated for clarity and convenience of explanation. In addition, terms specifically defined in consideration of the configuration and operation of the present disclosure may vary according to the intentions or customs of users and operators. Definitions of these terms should be made based on the content throughout this specification.

In the description of the present disclosure, it should be noted that the orientation or positional relationship indicated by the terms "center", "upper", "lower", "left", "right", "vertical", "horizontal", "inner side", "outer side", "one surface", "other surface" is based on the orientation or positional relationship shown in the drawing or the orientation or positional relationship normally arranged when using the product of the present disclosure, and it is intended only for explanation and brief description of the present disclosure, and is not to be construed as limiting the present disclosure as it does not suggest or imply that the device or element shown must necessarily be configured or operated in a specific orientation with a specific orientation.

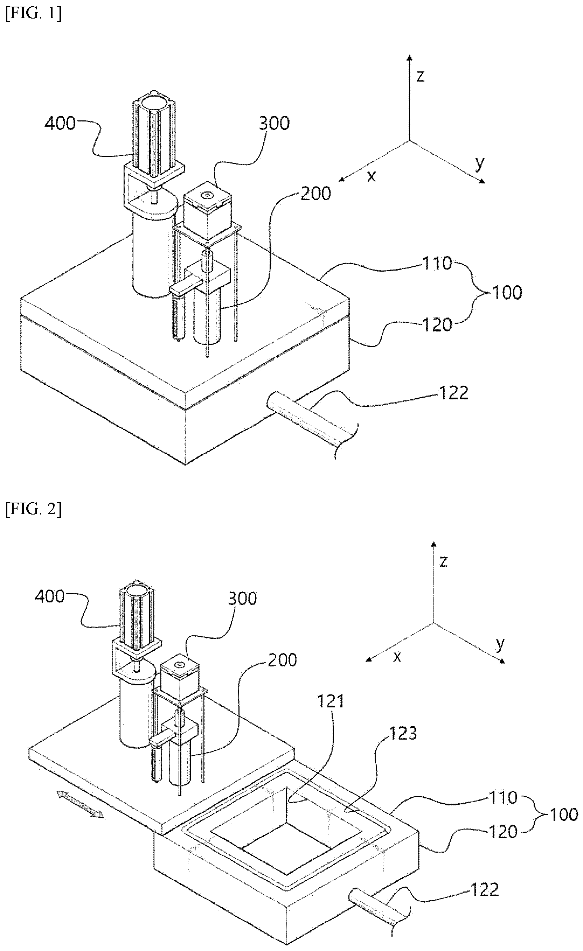

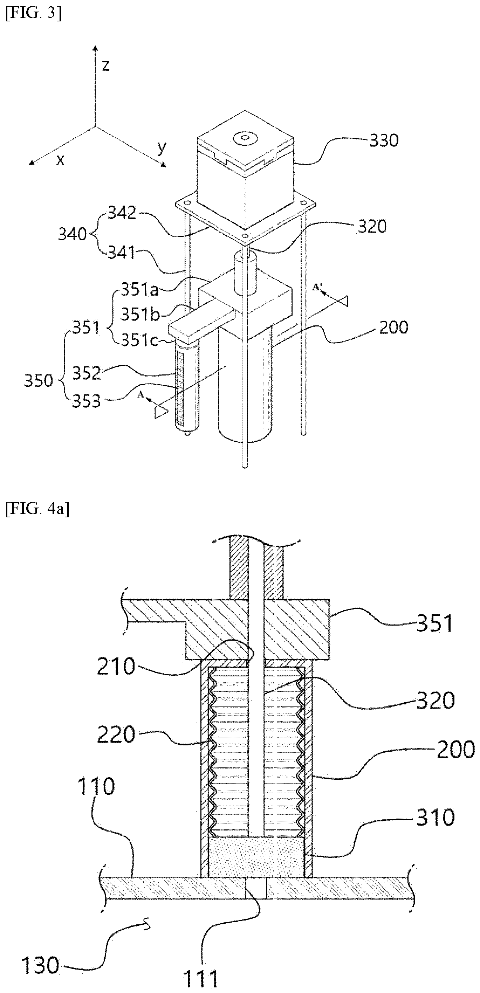

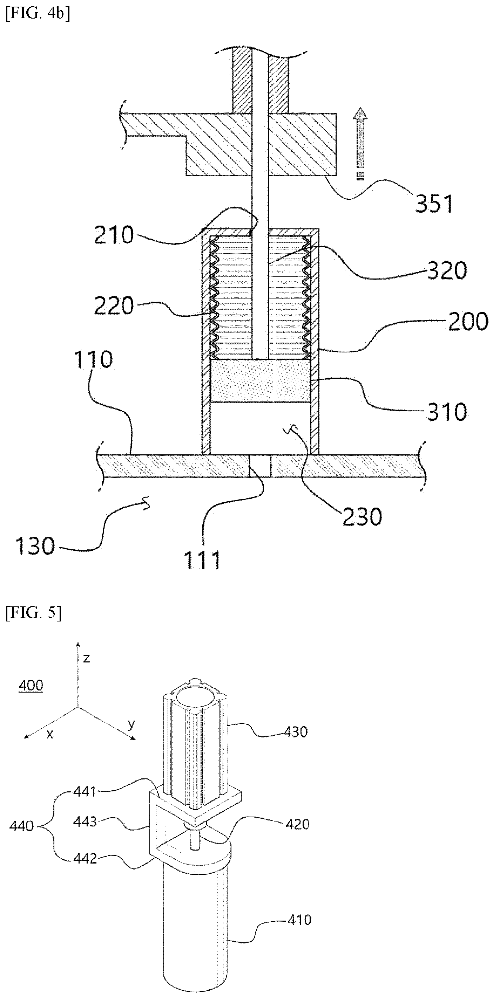

FIG. 1 is a perspective view illustrating a gas collecting apparatus according of the present disclosure. FIG. 2 is a perspective view illustrating a state in which a gas diffusion chamber 100 is opened. FIG. 3 is a perspective view illustrating a variable dilution tank 200 and a volume control unit 300. FIG. 4a is a cross-sectional view illustrating a cross section A-A' of FIG. 3. FIG. 4b is a cross-sectional view illustrating a state in which a dilution space 230 is expanded. FIG. 5 is a perspective view illustrating a punching unit 400.

Hereinafter, the gas collecting apparatus of the present disclosure will be described in detail with reference to FIGS. 1 to 5.

As shown in FIG. 1, the gas collecting apparatus of the present disclosure may include:
the gas diffusion chamber 100 provided with a gas diffusion space 130 inside;
the variable dilution tank 200 connected to the gas diffusion chamber 100 and provided with the dilution space 230 inside;
the volume control unit 300 configured to control a volume of the dilution space 230 of the variable dilution tank 200;
the punching unit 400 configured to form a perforated hole in a case of a battery accommodated in the gas diffusion space 130 of the gas diffusion chamber 100; and
a gas delivery flow path 122 connected to the gas diffusion chamber 100 to receive gas diffused in the gas diffusion space 130 and deliver the gas to a gas collecting container or a gas analyzer.

Gas capture using the gas capture apparatus of the present disclosure may be accomplished as follows. First, the battery is accommodated in the gas diffusion space 130 provided inside the gas diffusion chamber 100. A perforated hole is formed in the battery case through the punching unit 400 while the battery is accommodated in the gas diffusion space 130. When the gas generated inside the battery is discharged out of the battery through the perforated hole, the battery generated gas is diffused into the gas diffusion space 130 of the gas diffusion chamber 100. The pressure of the gas diffusion space 130 is measured, and when the pressure is greater than the set pressure, the volume control unit 300 is operated to expand the dilution space 230 of the variable dilution tank 200. When the pressure of the gas diffusion space 130 is lower than the set pressure, a gas collecting container or a gas analyzer may be connected to the gas delivery flow path 122 to collect the battery generated gas.

As shown in FIGS. 1 and 2, the gas diffusion chamber 100 may include a lower jig 120 with a battery accommodating groove 121 formed on an upper surface as the gas diffusion space 130, and an upper jig 110 coupled to an upper surface of the lower jig 120 while covering the battery accommodating groove 121.

The upper jig 110 may be provided in a plate shape, and may be coupled to the lower jig 120 such that a lower surface contacts the upper end of the lower jig 120. The variable dilution tank 200, the volume control unit 300, and the punching unit 400 may be fixed to the upper surface of the upper jig 110. A first hole 111 and a second hole (not shown) may be formed in the upper jig 110. The variable dilution tank 200 may be fixed to the upper surface of the upper jig 110 while covering the first hole 111 and the punching unit 400 may be fixed to the upper surface of the upper jig 110 while covering the second hole. The first hole 111 may be a passage through which the gas in the gas diffusion space 130 is delivered to the dilution space 230, and the second hole may be a passage through which the punching unit 400 inserts the punching needle into the gas diffusion space 130.

In other words, a first hole 111 is formed on an upper surface of the upper jig 110, a lower end of the variable dilution tank 200 is coupled to the upper surface of the upper jig 110 while covering the first hole 111, and gas in the gas diffusion space 130 and gas in the dilution space 230 are mutually ventilated through the first hole 111. At this time, the volume of the dilution space 230 is varied by the volume control unit 300, and concentration control of the gas diffused in the gas diffusion space 130 may be achieved by controlling the volume of the dilution space 230.

On the upper surface of the lower jig 120, a battery accommodating groove 121 provided to the gas diffusion space 130 may be provided. On the upper surface of the lower jig 120, a sealing member insertion groove 123 may be provided so that when the upper jig 110 and the lower jig 120 are coupled, the sealing member is inserted into the sealing member insertion groove 123 to enhance the airtightness of the gas diffusion space 130. The sealing member and the sealing member insertion groove 123 are provided in a closed loop shape, and the battery insertion groove may be located inside the sealing member insertion groove 123. For example, a sealing member may be an O-ring.

As shown in FIG. 2, the upper jig 110 may be coupled with or separated from the lower jig 120 while sliding in the y-axis direction. The upper jig 110 may linearly move in the y-axis direction by a pneumatic cylinder (not shown).

The gas delivery flow path 122 may be a chemical resistant pipe. The gas delivery flow path 122 may be provided with an opening/closing valve for discharging or blocking the gas in the gas diffusion space 130. A manifold may be connected to the gas delivery flow path 122, and a pressure sensor may be connected to the manifold to measure the pressure of the gas diffusion space 130.

As shown in FIGS. 3 and 4a, the volume control unit 300 may include a moving unit 310 which is located inside the variable dilution tank 200 and moves in a vertical direction, a shaft 320 coupled to an upper surface of the moving unit 310 in a state in which a lower end is inserted inside the variable dilution tank 200 through a penetration hole 210 provided on an upper surface of the variable dilution tank 200 and moving in the vertical direction, and a first driving unit 330 configured to rotate the shaft 320 with the vertical direction as a rotation axis.

The moving unit 310 may adjust the volume of the dilution space 230 by moving in the inner space of the variable dilution tank 200 while separating the inner space of the variable dilution tank 200 into two spaces.

Specifically, an inner space of the variable dilution tank 200 may be provided in a column shape extending in the vertical direction, a side surface of the moving unit 310 may be in close contact with an inner circumferential surface of the variable dilution tank 200 in the inner space of the variable dilution tank 200, and the dilution space 230 may be a space located at a lower side of the moving unit 310 in the inner space of the variable dilution tank 200. For example, the dilution space 230 and the moving unit 310 of the variable dilution tank 200 may be provided in a cylindrical shape extending in the vertical direction, and the outer circumferential surface of the moving unit 310 may be in close contact with the inner circumferential surface of the variable dilution tank 200.

As shown in FIG. 4b, when the moving unit 310 is raised by the volume control unit 300, the dilution space 230 of the variable dilution tank 200 may be expanded, and the gas concentration in the gas diffusion space 130 may be controlled. The gas collecting apparatus of the present disclosure may precisely control the movement of the moving unit 310 by converting rotational force into a linear driving force to move the moving unit 310.

As shown in FIGS. 4a and 4b, a sealing cover 220 surrounding the shaft 320 may be provided at an upper side of the moving unit 310 in the inner space of the variable dilution tank 200, and the sealing cover 220 may be contracted or expanded in the vertical direction. An upper end of the sealing cover 220 may be coupled to a ceiling surface of the variable dilution tank 200 in the inner space of the variable dilution tank 200, and a lower end of the sealing cover 220 may be coupled to the upper surface of the moving unit 310. The sealing cover 220 not only prevents gas leaking to the side of the moving unit 310 from being discharged to the outside through the penetration hole 210, but also may prevent gas from being diffused into spaces other than the dilution space 230 in the inner space of the variable dilution tank 200. For example, the outer circumferential surface of the sealing cover 220 is formed to be as close as possible to the inner circumferential surface of the variable dilution tank 200, so that even if the lower gas of the moving unit 310 leaks upward, the volume in which gas is substantially diffused inside the variable dilution tank 200 may be approximated as a volume of the dilution space 230. The upper and lower ends of the sealing cover 220 may be welded to a ceiling surface of the variable dilution tank 200 and the upper surface of the moving unit 310, respectively.

The shaft 320 may be provided in a cylindrical shape extending in the vertical direction and a screw thread may be formed on an outer circumferential surface of the shaft 320. The shaft 320 may be screw-coupled to the variable dilution tank 200 or a structure having a fixed location relative to the variable dilution tank 200. Accordingly, the shaft 320 may move in the vertical direction with respect to the variable dilution tank 200 by rotating. For example, a screw thread may be provided on the inner circumferential surface of the penetration hole 210, and by rotating the shaft 320 in a state where the thread of the penetration hole 210 and the thread of the shaft 320 are coupled, the shaft 320 may move in the vertical direction with respect to the variable dilution tank 200. The lower end of the shaft 320 is coupled to the moving unit 310 so as to be rotatable so that the moving unit 310 can be prevented from rotating together when the shaft 320 rotates.

The first driving unit 330 may rotate the shaft 320 with the vertical direction as a rotation axis. The first driving unit 330 may be an electric motor.

The volume control unit 300 may further include a support unit 340 for fixing a relative distance between the first driving unit 330 and the upper jig 110.

As shown in FIG. 3, the support unit 340 may include a support plate 342 located on the upper part of the variable dilution tank 200 and spaced apart from the upper end of the variable dilution tank 200, and a column member 341 having an upper end coupled to the lower surface of the support plate 342 and a lower end coupled to the upper surface of the upper jig 110.

The first driving unit 330 may be fixed to the upper surface of the support plate 342 and a hole may be formed in the support plate 342 so that the shaft 320 can penetrate the support plate 342. The shaft 320 may be screwed to the support plate 342. In this case, a screw thread may not be provided in the penetration hole 210.

As shown in FIG. 3, the volume control unit 300 may further include a measuring unit 350 configured to measure a moving distance of the moving unit 310 in the vertical direction.

The measuring unit 350 may include a moving member 351 configured to move in the vertical direction along with the shaft 320, a fixing member 352 coupled to the upper surface of the upper jig 110, and a display member 353 configured to indicate displacement between the moving member 351 and the fixing member 352.

The moving member 351 may be coupled to the shaft 320 so as to be rotatable, and may move in the vertical direction together with the shaft 320. For example, a stopper (not shown) may be provided on the shaft 320 so that the moving member 351 can move along with the shaft 320 in the vertical direction.

Specifically, the moving member 351 may include a first moving member 351a coupled to the shaft 320 so as to be rotatable, a second moving member 351b protruding from the side of the first moving member 351a, and a third moving member 351c having an upper end coupled to the second moving member 351b and a lower end inserted into the fixing member 352.

For example, the third moving member 351c and the fixing member 352 may be provided in a cylindrical shape, and the third moving member 351c may be inserted into the fixing member 352. A scale may be displayed on the display member 353 so that the moving distance of the moving member 351 can be measured.

As described above, on the upper surface of the upper jig 110, a second hole for inserting a punching needle into the gas diffusion space 130 may be formed, and as shown in FIG. 5, the punching unit 400 may include a punching needle housing 410 coupled to the upper surface of the upper jig 110 with a lower end covering the second hole and configured to accommodate the punching needle inside, a rod 420 with a lower end coupled to the punching needle inside the punching needle housing 410, a second driving unit 430 coupled to an upper end of the rod 420 and configured to move the rod 420 in the vertical direction, and a fixing unit 440 configured to fix a position between the punching needle housing 410 and the second driving unit 430.

The fixing unit 440 may include a first fixing plate 441 having an upper surface to which the lower end of the second driving unit 430 is coupled, a second fixing plate 442 having a lower surface to which the punching needle housing 410 is coupled, and a fixing column 443 fixing the first fixing plate 441 and the second fixing plate 442 such that the distance between the first fixing plate 441 and the second fixing plate 442 is maintained constant.

On the first fixing plate 441 and the second fixing plate 442, a hole through which the rod 420 is penetrated may be formed.

Although embodiments according to the present disclosure have been described above, they are only illustrative and those skilled in the art will understand that various modifications and embodiments of equivalent range are possible therefrom. Therefore, the true technical protection scope of the present disclosure should be defined by the following claims.

<Explanation of Symbols> 100...Gas diffusion chamber, 110...Upper jig, 111...First hole, 120...Lower jig, 121...Battery accommodating groove, 122...Gas delivery flow path, 130...Gas diffusion space, 200...Variable dilution tank, 210... Penetration hole, 220...Sealing cover, 230...Dilution space, 300...Volume control unit, 310...Moving unit, 320...Shaft, 330...First driving unit, 340... Support unit, 341...Column member, 342... Support plate, 350...Measuring unit, 351... Moving member, 351a...First moving member, 351b...Second moving member, 351c...Third moving member, 352...Fixing member, 353... Display member, 400...Punching unit, 410...Punching needle housing, 420...Rod, 430...Second driving unit, 440...Fixing unit, 441...First fixing plate, 442...Second fixing plate, 443...Fixing column

### Industrial Applicability

A gas collecting apparatus of the present disclosure may be capable of collecting a gas with a constant concentration in a gas collecting container by providing a controllable variable space to a gas diffusion space.

The gas collecting apparatus of the present disclosure may provide an optimized gas concentration to an analysis device by controlling a space in which gas generated from a battery is diffused in the process of collecting the gas.

## Claims

1. A gas collecting apparatus comprising:
a gas diffusion chamber (100) provided with a gas diffusion space (130) inside;
a variable dilution tank (200) connected to the gas diffusion chamber (100) and provided with a dilution space (230) inside;
a volume control unit (300) configured to control a volume of the dilution space (230) of the variable dilution tank (200);
a punching unit (400) configured to form a perforated hole in a case of a battery accommodated in the gas diffusion space (130) of the gas diffusion chamber (100); and
a gas delivery flow path (122) connected to the gas diffusion chamber (100) to receive gas diffused in the gas diffusion space (130) and deliver the gas to a gas collecting container or a gas analyzer, the gas collecting apparatus being **characterised in that** the volume control unit (300) is configured to be operated to expand the dilution space (230) of the variable dilution tank (200) when a pressure of the gas diffusion space (130) is greater than a set pressure.

2. The gas collecting apparatus of claim 1, wherein the volume control unit (300) comprises:
a moving unit (310) which is located inside the variable dilution tank (200) and moves in a vertical direction;
a shaft (320) which is coupled to an upper surface of the moving unit (310) in a state in which a lower end is inserted inside the variable dilution tank (200) through a penetration hole (210) provided on an upper surface of the variable dilution tank (200) and moves in the vertical direction; and
a first driving unit configured to rotate the shaft (320) with the vertical direction as a rotation axis.

3. The gas collecting apparatus of claim 2, wherein the gas diffusion chamber (100) comprises:
a lower jig (120) with a battery accommodating groove (121) formed on an upper surface as the gas diffusion space (130); and
an upper jig (110) coupled to an upper surface of the lower jig (120) while covering the battery accommodating groove (121), and
wherein a first hole (111) is formed on an upper surface of the upper jig (110),
a lower end of the variable dilution tank (200) is coupled to the upper surface of the upper jig (110) while covering the first hole (111), and
gas in the gas diffusion space (130) and gas in the dilution space (230) are mutually ventilated through the first hole (111).

4. The gas collecting apparatus of claim 3, wherein the volume control unit (300) further comprises a support unit (340) configured to fix a relative distance between the first driving unit and the upper jig (110).

5. The gas collecting apparatus of claim 3, wherein the volume control unit (300) further comprises a measuring unit (350) configured to measure a moving distance of the moving unit (310) in the vertical direction.

6. The gas collecting apparatus of claim 5, wherein the measuring unit (350) comprises:
a moving member (351) configured to move in the vertical direction along with the shaft (320);
a fixing member (352) coupled to the upper surface of the upper jig (110); and
a display member (353) configured to indicate displacement between the moving member (351) and the fixing member (352).

7. The gas collecting apparatus of claim 2, wherein an inner space of the variable dilution tank (200) is provided in a column shape extending in the vertical direction,
a side surface of the moving unit (310) is in close contact with an inner circumferential surface of the variable dilution tank (200), and
the dilution space (230) is a space located at a lower side of the moving unit (310) in the inner space of the variable dilution tank (200).

8. The gas collecting apparatus of claim 7, wherein a sealing cover (220) surrounding the shaft (320) is provided at an upper side of the moving unit (310) in the inner space of the variable dilution tank (200), and
the sealing cover (220) is contracted or expanded in the vertical direction.

9. The gas collecting apparatus of claim 8, wherein an upper end of the sealing cover (220) is coupled to a ceiling surface of the variable dilution tank (200) in the inner space of the variable dilution tank (200), and
a lower end of the sealing cover (220) is coupled to the upper surface of the moving unit (310).

10. The gas collecting apparatus of claim 3, wherein a second hole for inserting a punching needle into the gas diffusion space (130) is formed on the upper surface of the upper jig (110), and
the punching unit (400) comprises:
a punching needle housing (410) coupled to the upper surface of the upper jig (110) with a lower end covering the second hole and configured to accommodate the punching needle inside;
a rod (420) with a lower end coupled to the punching needle inside the punching needle housing (410);
a second driving unit (430) coupled to an upper end of the rod (420) and configured to move the rod (420) in the vertical direction; and
a fixing unit (440) configured to fix a position between the punching needle housing (410) and the second driving unit (430).

## Patentansprüche

1. Gassammelvorrichtung, umfassend:
eine Gasdiffusionskammer (100), die innen mit einem Gasdiffusionsraum (130) versehen ist
einen variablen Verdünnungsbehälter (200), der mit der Gasdiffusionskammer (100) verbunden und innen mit einem Verdünnungsraum (230) versehen ist;
eine Volumensteuereinheit (300), die dazu konfiguriert ein Volumen des Verdünnungsraums (230) des variablen Verdünnungsbehälters (200) zu steuern;
eine Schlageinheit (400), die dazu konfiguriert ist, eine Lochperforation in einem Gehäuse einer in dem Gasdiffusionsraum (130) der Gasdiffusionskammer (100) untergebrachten Batterie zu bilden; und
einen Gaszufuhrströmungsweg (122), der mit der Gasdiffusionskammer (100) verbunden ist, um in den Gasdiffusionsraum (130) diffundiertes Gas aufzunehmen und das Gas an einen Gassammelbehälter oder einen Gasanalysator zu liefern, wobei die Gassammelvorrichtung **dadurch gekennzeichnet ist, dass** die Volumensteuereinheit (300) so konfiguriert ist, dass sie betrieben wird, um den Verdünnungsraum (230) des variablen Verdünnungsbehälters (200) zu expandieren, wenn ein Druck des Gasdiffusionsraums (130) größer als ein eingestellter Druck ist.

2. Gassammelvorrichtung nach Anspruch 1, wobei die Volumensteuereinheit (300) umfasst:
eine Bewegungseinheit (310), die im Inneren des variablen Verdünnungsbehälters (200) angeordnet ist und sich in vertikaler Richtung bewegt;
eine Welle (320), die mit einer oberen Fläche der beweglichen Einheit (310) in einem Zustand gekoppelt ist, in dem ein unteres Ende in das Innere des variablen Verdünnungsbehälters (200) durch eine Durchgangsöffnung (210), die an einer oberen Fläche des variablen Verdünnungsbehälters (200) vorgesehen ist, eingeführt ist und sich in der vertikalen Richtung bewegt; und
eine erste Antriebseinheit, die so konfiguriert ist, dass sie die Welle (320) mit der vertikalen Richtung als Drehachse dreht.

3. Gassammelvorrichtung nach Anspruch 2, wobei die Gasdiffusionskammer (100) umfasst:
eine untere Halterung (120) mit einer Batterieaufnahmenut (121), die auf einer oberen Fläche als Gasdiffusionsraum (130) ausgebildet ist; und
eine obere Halterung (110), die mit einer oberen Fläche der unteren Halterung (120) verbunden ist, während sie die Batterieaufnahmenut (121) abdeckt, und
wobei eine erstes Öffnung (111) auf einer oberen Fläche der oberen Halterung (110) ausgebildet ist,
ein unteres Ende des variablen Verdünnungsbehälters (200) mit der oberen Fläche der oberen Halterung (110) gekoppelt ist, während es die erste Öffnung (111) abdeckt, und
Gas im Gasdiffusionsraum (130) und Gas im Verdünnungsraum (230) werden gleichsam durch die erste Öffnung (111) belüftet.

4. Gassammelvorrichtung nach Anspruch 3, wobei die Volumenkontrolleinheit (300) ferner eine Stützeinheit (340) umfasst, die so konfiguriert ist, dass sie einen relativen Abstand zwischen der ersten Antriebseinheit und der oberen Halterung (110) festlegt.

5. Gassammelvorrichtung nach Anspruch 3, wobei die Volumensteuereinheit (300) ferner eine Messeinheit (350) umfasst, die so konfiguriert ist, dass sie einen Bewegungsabstand der Bewegungseinheit (310) in der vertikalen Richtung misst.

6. Gassammelvorrichtung nach Anspruch 5, wobei die Messeinheit (350) umfasst:
ein bewegliches Element (351), das so konfiguriert ist, dass es sich in der vertikalen Richtung zusammen mit der Welle (320) bewegt;
ein Befestigungselement (352), das mit der oberen Fläche der oberen Halterung (110) verbunden ist; und
ein Anzeigeelement (353), das so konfiguriert ist, dass es die Verschiebung zwischen dem beweglichen Element (351) und dem Befestigungselement (352) anzeigt.

7. Gassammelvorrichtung nach Anspruch 2, wobei ein Innenraum des variablen Verdünnungstanks (200) in einer sich in vertikaler Richtung erstreckenden Säulenform vorgesehen ist,
eine Seitenfläche der beweglichen Einheit (310) in engem Kontakt mit einer inneren Umfangsfläche des variablen Verdünnungsbehälters (200) steht, und
der Verdünnungsraum (230) ein Raum ist, der sich an einer Unterseite der beweglichen Einheit (310) im Innenraum des variablen Verdünnungsbehälters (200) befindet.

8. Gassammelvorrichtung nach Anspruch 7, wobei eine Dichtungsabdeckung (220), die die Welle (320) umgibt, an einer Oberseite der beweglichen Einheit (310) im Innenraum des variablen Verdünnungsbehälters (200) vorgesehen ist, und
die Dichtungsabdeckung (220) in vertikaler Richtung zusammengezogen oder ausgedehnt wird.

9. Gassammelvorrichtung nach Anspruch 8, wobei ein oberes Ende der Dichtungsabdeckung (220) mit einer Deckenfläche des variablen Verdünnungsbehälters (200) im Innenraum des variablen Verdünnungsbehälters (200) verbunden ist, und
ein unteres Ende der Dichtungsabdeckung (220) mit der oberen Fläche der Bewegungseinheit (310) gekoppelt ist.

10. Gassammelvorrichtung nach Anspruch 3, wobei eine zweite Öffnung zum Einführen einer Stanznadel in den Gasdiffusionsraum (130) an der oberen Oberfläche der oberen Halterung (110) ausgebildet ist, und
die Stanzeinheit (400) umfasst:
ein Stanznadelgehäuse (410), das mit der oberen Fläche der oberen Halterung (110) verbunden ist, wobei ein unteres Ende die zweite Öffnung abdeckt und so konfiguriert ist, dass es die Stanznadel im Inneren aufnimmt;
eine Stange (420), deren unteres Ende mit der Stanznadel im Inneren des Stanznadelgehäuses (410) verbunden ist;
eine zweite Antriebseinheit (430), die mit einem oberen Ende der Stange (420) gekoppelt und so konfiguriert ist, dass sie die Stange (420) in der vertikalen Richtung bewegt; und
eine Fixiereinheit (440), die zum Fixieren einer Position zwischen dem Stanznadelgehäuse (410) und der zweiten Antriebseinheit (430) konfiguriert ist.

## Revendications

1. Appareil de collecte de gaz, comprenant :
une chambre de diffusion de gaz (100) munie d'un espace de diffusion de gaz (130) à l'intérieur ;
un réservoir de dilution variable (200) relié à la chambre de diffusion de gaz (100) et pourvu d'un espace de dilution (230) à l'intérieur ;
un module régulateur du volume (300) configuré pour réguler un volume de l'espace de dilution (230) du réservoir de dilution variable (200) ;
un dispositif de poinçonnage (400) configuré pour former un orifice perforé dans le boîtier d'une batterie installée dans l'espace de diffusion de gaz (130) de la chambre de diffusion de gaz (100) ; et
un chemin d'écoulement de distribution de gaz (122) relié à la chambre de diffusion de gaz (100) pour recevoir le gaz diffusé dans l'espace de diffusion de gaz (130) et distribuer le gaz à un récipient de collecte de gaz ou à un analyseur de gaz, l'appareil de collecte de gaz étant **caractérisé en ce que** le module régulateur du volume (300) est configuré pour être actionné pour dilater l'espace de dilution (230) du réservoir de dilution variable (200) lorsqu'une pression de l'espace de diffusion de gaz (130) est supérieure à une pression de consigne.

2. Dispositif de collecte de gaz selon la revendication 1, le module régulateur du volume (300) comprenant :
une unité mobile (310) située à l'intérieur du réservoir à dilution variable (200) et se déplaçant dans le sens vertical ;
un arbre (320) couplé à une surface supérieure de l'unité mobile (310) dans un état dans lequel une extrémité inférieure est insérée à l'intérieur du réservoir à dilution variable (200) à travers un orifice de pénétration (210) agencé sur une surface supérieure du réservoir à dilution variable (200) et se déplaçant dans le sens vertical ; et
une première unité motrice configurée pour faire tourner l'arbre (320) avec le sens vertical en tant qu'axe de rotation.

3. Dispositif de collecte de gaz selon la revendication 2, la chambre de diffusion de gaz (100) comprenant :
un bâti inférieur (120) avec une rainure (121) recevant une batterie formée sur une surface supérieure comme l'espace de diffusion de gaz (130) ; et
un bâti supérieur (110) couplé à une surface supérieure du bâti inférieur (120) tout en couvrant la rainure recevant la batterie (121), et
un premier orifice (111) étant formé sur une surface supérieure du bâti supérieur (110),
une extrémité inférieure du réservoir à dilution variable (200) étant couplée à la surface supérieure du bâti supérieur (110) tout en couvrant le premier orifice (111), et
le gaz dans l'espace de diffusion de gaz (130) et le gaz dans l'espace de dilution (230) étant mutuellement ventilés à travers le premier orifice (111).

4. Appareil de collecte de gaz selon la revendication 3, le module de régulation du volume (300) comprenant en outre un module de support (340) configuré pour fixer une distance relative entre la première unité motrice et le bâti supérieur (110).

5. Appareil de collecte de gaz selon la revendication 3, le module de régulation du volume (300) comprenant en outre un dispositif de mesure (350) configuré pour mesurer une distance de déplacement de l'unité motrice (310) dans le sens vertical.

6. Dispositif de collecte de gaz selon la revendication 5, le dispositif de mesure (350) comprenant :
un élément mobile (351) configuré pour se déplacer dans le sens vertical conjointement avec l'arbre (320) ;
un élément de fixation (352) couplé à la surface supérieure du bâti supérieur (110) ; et
un élément d'affichage (353) configuré pour indiquer le déplacement entre l'élément mobile (351) et l'élément de fixation (352).

7. Appareil de collecte de gaz selon la revendication 2, un espace intérieur du réservoir à dilution variable (200) étant agencé sous forme d'une colonne s'étendant dans le sens vertical,
une surface latérale de l'unité mobile (310) étant en contact étroit avec une surface circonférentielle intérieure du réservoir à dilution variable (200), et
l'espace de dilution (230) étant un espace situé sur un côté inférieur de l'unité mobile (310) dans l'espace intérieur du réservoir à dilution variable (200).

8. Appareil de collecte de gaz selon la revendication 7, un couvercle d'étanchéité (220) entourant l'arbre (320) étant agencé sur un côté supérieur de l'unité mobile (310) dans l'espace intérieur du réservoir à dilution variable (200), et
le couvercle d'étanchéité (220) se contractant ou se détendant dans le sens vertical.

9. Appareil de collecte de gaz selon la revendication 8, une extrémité supérieure du couvercle d'étanchéité (220) étant couplée à une surface de plafond du réservoir à dilution variable (200) dans l'espace intérieur du réservoir à dilution variable (200), et
une extrémité inférieure du couvercle d'étanchéité (220) étant couplée à la surface supérieure de l'unité mobile (310).

10. Dispositif de collecte de gaz selon la revendication 3, un deuxième orifice pour l'insertion d'une aiguille de poinçonnage dans l'espace de diffusion de gaz (130) étant formé sur la surface supérieure du bâti supérieur (110), et
le dispositif de poinçonnage (400) comprenant :
un boîtier d'aiguille de poinçonnage (410) couplé à la surface supérieure du bâti supérieur (110), une extrémité inférieure couvrant le deuxième orifice et étant configurée pour recevoir l'aiguille de poinçonnage à l'intérieur ;
une tige (420) avec une extrémité inférieure couplée à l'aiguille de poinçonnage à l'intérieur du boîtier de l'aiguille de poinçonnage (410) ;
une deuxième unité motrice (430) couplée à une extrémité supérieure de la tige (420), et configurée pour déplacer la tige (420) dans le sens vertical ; et
un dispositif de fixation (440) configuré pour fixer une position entre le boîtier de l'aiguille de poinçonnage (410) et la deuxième unité motrice (430).
